# EUROPÄISCHE PATENTANMELDUNG

(11) **EP 3 524 983 A1**
(43) Veröffentlichungstag der Anmeldung: **14.08.2019**
(21) Anmeldenummer: 18000114.1
(22) Anmeldetag: 08.02.2018
(51) Int. Cl.: G01N 33/569, G01N 33/68

(54) **VERFAHREN ZUR QUANTIFIZIERUNG VON GESAMTGLUTEN AUS GETREIDE IN LEBENSMITTELPROBEN**

(71) Anmelder: R-Biopharm Aktiengesellschaft, 64297 Darmstadt (DE)
(72) Erfinder: Weiss, Thomas, 64291 Darmstadt (DE); Lacorn, Markus, 65375 Oestrich-Winkel (DE); Hektor, Thomas, 64347 Griesheim (DE)
(74) Vertreter: Baumbach, Friedrich

(57) **Zusammenfassung**

Die Erfindung betrifft ein Verfahren zur Quantifizierung von Gesamtgluten aus Getreide in Lebensmittelproben. Anwendungsgebiete sind vor allem die Lebensmittelindustrie, Servicelabore und staatliche Kontrolllabore oder die Biotechnologie.

Die Erfindung hat das Ziel, den Gesamt-Glutengehalt in Lebensmitteln mit nur einer Messung schnell und preiswert zu bestimmen. Daraus leitet sich die Aufgabe der vorliegenden Erfindung ab, ein Verfahren zu entwickeln, mit dem, basierend auf der Detektion von Prolaminen und Glutelinen, alle potentiell Zöliakie-relevanten Gluten-Proteinfraktionen in Lebensmittelproben entsprechend ihren Massen als Summe quantifiziert werden.

Das vorliegende Verfahren basiert auf der gemeinsamen Verwendung spezifischer Prolamin- und Glutelinantikörper als erfindungsgemäßes Kombinationskonjugat zur Detektion von Gesamtgluten. Dabei erfolgt eine Verdünnung der einzelnen Antikörperkonjugate derart, dass der Beitrag der einzelnen Antikörper zur Gesamtsignalstärke dem Anteil der Gluten-Fraktion entspricht, die sie jeweils detektieren.

## Beschreibung

### Gebiet der Erfindung

Die Erfindung betrifft ein Verfahren zur Quantifizierung von Gesamtgluten aus Getreide in Lebensmittelproben. Anwendungsgebiete sind vor allem die Lebensmittelindustrie, Servicelabore und staatliche Kontrolllabore oder die Biotechnologie.

### Hintergrund der Erfindung

Mit einer Jahresernte von 2,5 Milliarden Tonnen ist Getreide das wichtigste Nahrungsmittel des Menschen. Die Bandbreite der Lebensmittel, die aus Getreide hergestellt, mit Getreideprodukten ergänzt oder mit Getreideinhaltsstoffen behandelt werden, ist unüberschaubar. Einige Getreide, wie z.B. Weizen, Gerste und Roggen, enthalten große Mengen Klebereiweiße, das sogenannte Gluten, das zwar für den Backvorgang vorteilhaft ist, bei bestimmten Personen jedoch eine Unverträglichkeit auslöst, die man als Zöliakie bezeichnet. Zusätzlich wurde eine weitere Unverträglichkeit in Form der "Non Celiac Gluten Sensitivity" (nicht zöliakieassoziierte Glutensensitivität) beobachtet, die jedoch noch nicht vollständig erforscht ist.

Die Prävalenz der Zöliakie schwankt je nach Kulturkreis; im weltweiten Durchschnitt liegt sie bei 1:270, das sind also ca. 250 Mio. Menschen. Die Zöliakie ist eine teilweise autoimmune Krankheit, die zu einer Schädigung des Dünndarms führt, was sich in Durchfall, Erbrechen, Mangelernährungserscheinungen und Gewichtsverlust manifestiert. Da die Krankheit nicht heilbar ist, bleibt für den Erkrankten nur die strikte Gluten-freie Diät. Zöliakie-Patienten, die sich an eine solche Diät halten, leben weitgehend symptomfrei. Die Erkrankten sind somit auf Lebensmittel angewiesen, die kein oder sehr wenig Gluten enthalten.

Der 1963 durch die World Health Organization (WHO) und Food and Agricultural Organization of the United Nations (FAO) ins Leben gerufene Codex Alimentarius beschreibt die Anforderung an Lebensmittel, die als "Gluten-frei" gelten. Die aktuell gültigen Standards definieren Lebensmittel als "Gluten-frei", wenn sie weniger als 20 mg Gluten pro kg Lebensmittel enthalten (Codex Standard 118 - 1979). Andere Länder und Regionen wie z. B. die EU, USA und Kanada haben ihre gesetzlichen Verordnungen an diesen Codex-Grenzwert übernommen. Lebensmittelhersteller, die die Versorgung von Zöliakie-Patienten mit "Gluten-freien" Produkten gewährleisten möchten, müssen diesen Grenzwert für ihre Produkte überprüfen und einhalten.

Gluten ist ein heterogenes Proteingemisch aus alkohollöslichen Prolaminen und alkoholunlöslichen Glutelinen. Glutenproteine kommen in allen Triticum-Spezies (z. B. Weizen, Dinkel, Emmer, Einkorn), Roggen und Gerste vor, sowie in deren Kreuzungen und werden je nach Getreideart unterschieden. So werden im Weizen die Prolamine als Gliadine bezeichnet und die Gluteline in Low Molecular Weight Glutenin Subunit (LMW)-Proteine und High Molecular Weight Glutenin Subunit (HMW)-Proteine unterteilt. Historisch wurden nur die Prolamine als "toxisch" für Zöliakiepatienten angesehen. In letzter Zeit wurde jedoch festgestellt, dass auch LMW- und HMW- Proteine toxisch wirken (Tye-Din et al. 2010).

### Stand der Technik

Die am meisten verbreitete Detektionsmethode zum Nachweis von Glutenproteinen ist momentan der Enzyme-Linked-Immunosorbent-Assay (ELISA). Für die Extraktion der Lebensmittelproben werden Extraktionslösungen benutzt, die reduzierende Substanzen (z. B. β-Mercaptoethanol, Sulfit, Thiosulfit), denaturierende Substanzen (z. B. Guanidin-Hydrochlorid, Harnstoff) und/oder Detergenzien (z. B. Tween, Triton-X100, Natriumdodecylsulfat) enthalten. Eine dieser Extraktionsmethoden ist die sog. Cocktail-Extraktionslösung (EP 1 424 345 A1; Lizenz bei R-Biopharm AG). Alle Extraktionslösungen werden zusammen mit Ethanol oder Isopropanol verwendet.

Kommerziell erhältliche immunologische Detektionssysteme basieren meist auf monoklonalen Antikörpern (z. B. Skerritt, R5, G12 und Alpha20). Die meisten dieser Antikörper detektieren hauptsächlich oder ausschließlich die besser löslichen Prolamine (Wieser et al. 2014).

Beispielhaft wird nachfolgend eines der am häufigsten verwendeten Messsysteme zur Quantifizierung von Gluten näher erläutert, das sogenannten Mendez System bestehend aus Cocktail Extraktion und R5 ELISA. Dieses System ist z. B. kommerziell erhältlich als Cocktail (patented) R7006 (R-Biopharm AG, Darmstadt, Deutschland) und RIDASCREEN® Gliadin R7001 (R-Biopharm AG). Die homogenisierte Lebensmittelprobe wird mittels Cocktail (patented) bei 50°C extrahiert und anschließend mit Ethanol versetzt. Nach Verdünnung des erhaltenen Extraktes wird die so vorbereitete Probe auf einer, mit dem monoklonalen Antikörper R5, beschichteter Mikrotiterplatte inkubiert. Nach Waschen wird ein R5-Meerrettichperoxidase-Konjugat inkubiert. Nach einem weiteren Waschschritt erfolgt die Farbreaktion durch Zusatz einer Substrat/Chromogen Lösung. Die Reaktion wird durch Schwefelsäure gestoppt und die optische Dichte (OD) mittels Photometer gemessen. Die Quantifizierung erfolgt anhand mitgeführter Kalibratoren bekannten Gliadingehaltes. Aus dem so erhaltenen Gliadin-Gehalt wird durch Multiplikation mit dem Faktor 2 der Gesamt-Glutengehalt errechnet.

Diese Multiplikation ist gemäß Codex-Alimentarius vorgeschrieben (CODEX-STAN 118 - 1979). Zunehmend wird jedoch deutlich, dass ein allgemeiner Faktor 2 in vielen Fällen zu einer Fehlkalkulation führt (Wieser und Köhler 2009). So können sich z. B. Weizenmehle sowohl im Gesamtproteingehalt als auch in der Zusammensetzung der Glutenproteine deutlich unterscheiden (Hajas et al., 2017). Wird allein der Prolamin-Gehalt bestimmt, so kann dies bei unterschiedlicher Zusammensetzung der einzelnen Glutenproteinfraktionen zu verfälschten Ergebnissen bei der Berechnung des Gesamt-Glutengehalts führen.

Weiterhin können je nach Aufbereitung des Getreides zum "Gluten-freien" Produkt bestimmte Glutenfraktionen (Prolamine und Gluteline) selektiv ab- oder angereichert werden, so dass bei einer nur teilweisen Bestimmung der Glutenfraktionen es zu signifikanten Fehlbestimmungen kommen kann (Wieser und Köhler 2009).

Ein gemeinsamer Nachweis von Prolaminen und Glutelinen, der diese Probleme beheben könnte, wird im Stand der Technik nicht beschrieben. So offenbart beispielsweise EP 1 1779 115 B1 verschiedene Antikörper gegen T-Zell Erkennungssequenzen in Gliadin (Prolamin in Weizen) bzw. in LMW und HMW (Gluteline in Weizen) und deren Verwendung in ELISA-Assays. Allerdings erfolgt kein gemeinsamer Nachweis, sondern nur entweder ein Nachweis von Prolamin oder LMW oder HMW. Der getrennte Nachweis wird auch als besonders vorteilhaft hervorgehoben.

Wenige kommerzielle ELISA Assays verwenden polyklonale Antikörper (Wieser et al. 2014). Selbst bei Immunisierung mit Gesamtgluten wird die Antikörper-Reaktivität zufällig erzeugt. Dadurch ist es nicht möglich, im Nachhinein eine Einstellung des Systems vorzunehmen, so dass jede Glutenproteinfraktion entsprechend ihres Masseanteils quantifiziert werden kann.

Aufgrund der genannten Nachteile sind diese Methoden nicht mehr Mittel der Wahl, um den tatsächlichen und genauen Gesamtgehalt von Glutenproteinen in Lebensmittel zu bestimmen.

### Ziel und Aufgabe der Erfindung

Die Erfindung hat das Ziel, den Gesamt-Glutengehalt in Lebensmitteln mit nur einer Messung schnell und preiswert zu bestimmen, wobei jede Glutenproteinfraktion entsprechend ihres Massenanteils quantifiziert werden soll.

Daraus leitet sich die Aufgabe der vorliegenden Erfindung ab, ein Verfahren zu entwickeln, mit dem, basierend auf der Detektion von Prolaminen und Glutelinen, alle potentiell Zöliakie-relevanten Gluten-Proteinfraktionen in Lebensmittelproben entsprechend ihren Massen als Summe quantifiziert werden. Das Verfahren soll zur Bestimmung des Gesamt-Glutengehaltes aus Weizenproben genutzt werden. Verwandte Glutenfraktionen in anderen Triticum-Spezies, Roggen und Gerste sollen mit dem Verfahren ebenfalls quantifizierbar sein.

Diese Aufgabe wird durch ein Verfahren gemäß Anspruch 1 gelöst. Weitere mögliche Ausführungsformen ergeben sich aus den Unteransprüchen, der Beschreibung und den Beispielen.

### Wesen der Erfindung

Das vorliegende Verfahren basiert auf der gemeinsamen Verwendung spezifischer Prolamin- und Glutelinantikörper als erfindungsgemäßes Kombinationskonjugat zur Detektion von Gesamtgluten. Dabei erfolgt eine Verdünnung der einzelnen Antikörperkonjugate derart, dass der Beitrag der einzelnen Antikörper zur Gesamtsignalstärke dem Anteil der Gluten-Fraktion entspricht, die sie jeweils detektieren.

### Kurzbeschreibung des Verfahrens:

Der Gesamt-Glutenbestimmung vorgeschaltet ist die Extraktion der Glutenproteine aus dem Lebensmittel. Dazu wird eine Extraktionsmethode verwendet, die unter reduktiven Bedingungen unter Zusatz von chaotropen Salzen in Anwesenheit von aliphatischen kurzkettigen Alkoholen, Glutenproteine aus der Lebensmittelmatrix herauslöst.

Der erhaltene Extrakt wird anschließend zur Gesamt-Glutenbestimmung in einem antikörperbasierten Nachweisverfahren z. B. in einem ELISA (Sandwich oder kompetitives oder direktes Format) eingesetzt.

Zum besseren Verständnis wird nachfolgend das Prinzip eines Sandwich-ELISAs erläutert. Bei einem Sandwich-ELISA wird das Antigen (Analyt) sandwichartig von zwei Antikörpern spezifisch gebunden. Der erste Antikörper (Fänger-Antikörper) wird an die feste Phase der Vertiefungen (Kavitäten) einer Mikrotiterplatte gebunden. Die Probe mit dem nachzuweisenden Antigen wird dann in die Kavitäten gegeben und inkubiert. Während dieser Zeit bindet der an die Platte gebundene Antikörper das in der Probe vorhandene Antigen. Nach Ablauf der Inkubationsphase wird die Platte gewaschen: Die ungebundenen Bestandteile der Probe werden dadurch entfernt. Zurück bleibt nur das am Fänger-Antikörper gebundene Antigen. Im nächsten Schritt wird ein zweiter Antikörper zur Detektion (Detektionsantikörper) zugegeben, der an eine andere Stelle im Antigen als der Fänger-Antikörper bindet und an dessen Ende ein Reporterenzym gebunden ist. Dieser zweite Antikörper bindet also ebenfalls an das Antigen, nur an einer anderen Position, und es entsteht ein Antikörper-Antigen-Antikörper-Komplex, der auch als Sandwich-Komplex bezeichnet wird. Durch erneutes Waschen der Platte wird überschüssiger, nicht gebundener Detektionsantikörper entfernt. Anschließend kann das Antigen detektiert und quantifiziert werden: Es wird ein zum Reporterenzym passendes Substrat (auch "Chromogen" genannt) zugegeben, das vom Reporterenzym zu einem Reaktionsprodukt umgesetzt wird und auf diese Weise dessen Nachweis durch Farbumschlag ermöglicht. Weitere Möglichkeiten anstelle des Reporterenzyms wären direkte Fluoreszenz- oder Chemolumineszenzmarkierung des Detektorantikörpers. Für eine quantitative Bestimmung wird üblicherweise eine Serie mit bekannten Antigenkonzentrationen (*Standardreihe*) mitgeführt, um eine Kalibrationskurve für das gemessene Signal zu erhalten.

Im Fall der vorliegenden Erfindung wird zur Durchführung eines Sandwich-ELISAs vor der Assay-Durchführung eine Mikrotiterplatte mit einer Kombination aus Antikörpern gegen Weizenprolamin, LMW- und HMW-Proteine beschichtet und anschließend die extrahierte Probe zugegeben. Die extrahierten Gluten-Proteine binden spezifisch an diese Fänger-Antikörper. Nach einem Waschschritt erfolgt die erfindungsgemäße kombinierte Zugabe von Detektions-Antikörpern gegen Weizenprolamin, LMW- und HMW-Proteine (Kombinationskonjugat). Diese Detektionsantikörper sind so konjugiert, dass deren Bindung quantifizierbar wird. Dies kann z. B. durch Konjugation mit einem farbstoffbildenden Reporterenzymvorgenommen werden. Darüber hinaus werden die Antikörper erfindungsgemäß in solchen Mengenverhältnissen eingesetzt, dass ihr Beitrag zur Gesamtsignalstärke dem Anteil der Gluten-Fraktion, die sie detektieren, in der Getreideprobe entspricht.

Die Quantifizierung erfolgt durch Vergleich der Gesamtsignalstärke einer Probe mit den Signalstärken von Glutenkalibratoren bekannten Gehaltes. Als Kalibratormaterial kann z. B. ein Extrakt aus Weizenmehl verwendet werden.

Die Erfindung wird nachfolgend mit konkreten Ausführungsbeispielen näher erläutert, welche jedoch nicht beschränkend für den Schutzumfang der Erfindung sein sollen. Für einen Fachmann naheliegende Abwandlungen und Änderungen der Erfindung fallen dementsprechend unter den Schutzumfang der Patentansprüche.

Wie bereits in einem der oberen Abschnitte erwähnt, ist der Gesamt-Glutenbestimmung die Extraktion der Glutenproteine aus der zu untersuchenden Probe unter Zusatz von chaotropen Salzen in Anwesenheit von aliphatischen kurzkettigen Alkoholen vorgeschaltet. Beispielhaft wird nachfolgend die Extraktion von Weizen-Glutenproteinen aus Lebensmittelproben verschiedenster Art mit einer Cocktail-Extraktionslösung [0,76 g/L NaCl, 0,224 g/L KCl, 1,42 g/L Na₂HPO₄*2H₂O, 0,272 g/L H₂KO₄P, 191 g/L Guanidin-HCI, 18,2 ml/L β-Mercaptoethanol] (EP 1 424 345 A1) stichpunktartig beschrieben.
- Eine ausreichend große Menge der Probe (mind. 5 g bzw. 5 ml) gut homogenisieren (sorgfältig zerstoßen, fein zermahlen und gut mischen bzw. die Lösung gut mischen).
- bei flüssigen Proben: zu 0,25 ml der homogenisierten Probe 2,5 ml Cocktail-Extraktionslösung (EP 1 424 345 A1) hinzugeben, Gefäß verschließen und gut mischen
- sonstige Proben (z.B. soja- und quinoahaltige Lebensmittelproben): 0,25 g der homogenisierten Probe einwiegen und 2,5 ml Cocktail-Extraktionslösung (EP 1 424 345 A1) hinzugeben, Gefäß verschließen und gut mischen
- tannin- und polyphenolhaltige Lebensmittelproben: (z.B. Schokolade, Kaffee, Kakao, Kastanienmehl, Buchweizen, Hirse und Gewürzen): 0,25 g der homogenisierten Probe einwiegen, 0,25 g Magermilchpulver und 2,5 ml Cocktail-Extraktionslösung (EP 1 424 345 A1) hinzugeben, Gefäß verschließen und gut mischen
- Fleisch- und Wurstwarenproben: die Glutenverteilung kann in diesen Lebensmitteln sehr ungleich sein, deshalb 50 g Probe einwiegen und homogenisieren: 0,25 g der homogenisierten Probe einwiegen und 2,5 ml Cocktail-Extraktionslösung (patented) hinzugeben, Gefäß verschließen und gut mischen
- Haferproben: die Glutenverteilung kann sehr ungleich sein, zusätzlich sind diese Proben schwer zu homogenisieren. Deshalb 200 g Probe homogenisieren, die Probenaufarbeitung sollte dann mindestens mit dem vierfachen Ansatz durchgeführt werden: 1 g der homogenisierten Probe einwiegen und 10 ml Cocktail-Extraktionslösung (patented) hinzugeben, Gefäß verschließen und gut mischen.

Alle Proben werden wie im Folgenden beschrieben weiter behandelt:
- 40 min bei 50 °C inkubieren
- Probe abkühlen lassen und anschließend mit 7,5 ml 80 % Ethanol versetzen (bei Haferproben: 30 ml 80 % Ethanol)
- Gefäß verschließen und 1 h bei Raumtemperatur (20 - 25 °C) über Kopf schütteln oder mittels Rotator rotieren lassen
- zentrifugieren: 10 min, mind. 2500 g, bei Raumtemperatur (20 - 25 °C) und/oder filtrieren (alternativ 2 ml des Extraktes in ein Reaktionsgefäß überführen und in einer Mikrozentrifuge für 10 min hochtourig zentrifugieren)
- den Überstand in ein verschließbares Röhrchen überführen
- die Probe 1:25 (40 µl + 960 µl) mit Puffer, z. B. pH neutralem Phosphatpufferweiter verdünnen: der finale Verdünnungsfaktor ist 1000

Die so erhaltene Probe wird anschließend zur Gesamt-Glutenbestimmung vorzugsweise in einem Sandwich-ELISA eingesetzt. Nachfolgend wird beispielhaft die Durchführung eines Sandwich-ELISAs zur Bestimmung von Gesamt-Gluten von Weizenhaltigen Proben beschrieben

Dazu werden zunächst die Kavitäten einer handelsübliche Mikrotiterplatte mit spezifischen Fänger-Antikörpern gegen Gliadin (Weizenprolamin), LMW- und HMW-Glutenin (Weizengluteline) beschichtet; typischerweise mit Konzentrationen zwischen 0,5 und 5 µg/ml Unspezifische Bindungen werden durch, aus der Literatur bekannten, Block- und Stabilisierungslösungen abgesättigt und anschließend die extrahierte Probe bzw. der Standard (Kalibrator) vorzugsweise als Doppelproben in die einzelnen Kavitäten zugegeben. Für die Herstellung des Kalibrators werden die Glutenproteine aus einem Weizenmehl mittels modifizierte Osborne Fraktionierung isoliert (Schalk et al. 2017). Zur Verbesserung der Haltbarkeit wird das so erhaltene Glutenisolat zur Trockne gebracht und gelagert. Zum Ansetzen der Kalibratoren wird dieses Isolat eingewogen, mittels Cocktail-Extraktionslösung (EP 1 424 345 A1) R-Biopharm (Produktnummer R7006) gelöst und in einem pH neutralem Phosphatpuffer auf die gewünschten Konzentrationen verdünnt.

Nach erfolgter Proben- bzw. Kalibratorzugabe und einem anschließenden Waschschritt mit einem gängigen Waschpuffer (z.B. PBS/Tween) erfolgt die kombinierte Zugabe von Detektions-Antikörpern gegen Gliadin (Weizenprolamin), LMW- und HMW-Glutenin (Weizengluteline), die alle jeweils mit einem Reporterenzym konjugiert sind. Dieses Gemisch von Detektionsantikörpern, die an anderen Stellen der Glutenproteine binden als die Fängerantikörper, wird nachfolgend als Kombinationskonjugat bezeichnet. (detaillierte Beschreibung der Herstellung des Kombinationskonjugats im folgenden Abschnitt). Nach anschließender Inkubation werden die einzelnen Kavitäten geleert, gewaschen, mit Substratreagenz aufgefüllt und für die Quantifizierung des Glutengehalts, also die quantitative Bestimmung des Anteils der an die Detektionsantikörper gebundenen Glutenproteine, durch Messung des Substratumsatzes durch die an die Detektionsantikörper gekoppelten Reporterenzyme bestimmt.

Das oben näher erläuterte Ausführungsverfahren mit einem *Sandwich ELISA* ist ein mögliches Verfahren. Weitere mögliche Verfahren sind z. B. ein *kompetitiver ELISA* (Antigenbeschichtung oder Antikörperbeschichtung) sowie ein *direkter ELISA.* Diese Verfahren werden im folgenden kurz beschrieben.

Bei einem *kompetitiven ELISA mit Antigenbeschichtung* wird die Mikrotiterplatte mit einem Glutenextrakt, z. B. aus einem Weizenmehl beschichtet. Der Kalibrator ist identisch mit dem beim Sandwich-ELISA-Verfahren. Die Herstellung sowie die Einstellung der Einzelkonjugate und des Kombinationskonjugates erfolgen wie beim Sandwich-ELISA-Verfahren, allerdings nimmt die OD bei einem kompetitiven ELISA bei zunehmenden Glutenkonzentrationen ab. Die Probenextraktion ist identisch mit der beim Sandwich-ELISA-Verfahren.

Bei der ELISA-Testdurchführung werden im ersten Schritt Kalibratoren bzw. Proben in die Mikrotiterplattenvertiefungen gegeben und direkt anschließend wird das Kombinationskonjugat zugegeben und Kalibrator bzw. Proben und Konjugat werden gemeinsam inkubiert. Allerdings ist die Meerrettich-Peroxidase nicht stabil gegenüber der Cocktail Extraktionslösung in den Proben. Daher müssen die Antikörper in diesem Fall anders konjugiert werden, z. B. an Biotin. Nach einem weiteren Waschschritt erfolgt die Detektion über Meerrettich-Peroxidase gekoppeltes Streptavidin. Alternativ werden unkonjugierte Antikörper verwendet und nach dem Waschschritt erfolgt die Detektion über Meerrettich-Peroxidase gekoppelten Sekundärantikörper. In beiden Fällen erfolgt nach einem erneuten Waschschritt die Zugabe des Farbreagenz. Im letzten Schritt wird die Reaktion durch Zugabe der Stopp-Lösung beendet.

Bei einem *kompetitiven ELISA mit Antikörperbeschichtung* wird die Mikrotiterplatte mit einer Lösung von Prolamin-, LMW- und HMW-Antikörper beschichtet. Der Kalibrator ist identisch mit dem beim Sandwich-ELISA-Verfahren. Als Konjugate werden die Einzelproteinfraktionen Prolamine, LMW und HMW an Meerrettich-Peroxidase gekoppelt. Die Einstellung der Einzelkonjugate und des Kombinationskonjugates erfolgen analog zum Sandwich-ELISA-Verfahren, allerdings nimmt die OD bei einem kompetitiven ELISA bei zunehmenden Glutenkonzentrationen ab. Die Probenextraktion ist identisch mit der beim Sandwich-ELISA-Verfahren.

Bei der ELISA-Testdurchführung werden im ersten Schritt Kalibratoren bzw. Proben in die Kavitäten gegeben und inkubiert. Nach einem Waschschritt wird das Kombinationskonjugat in die Kavitäten pipettiert und inkubiert. Nach einem erneuten Waschschritt wird das Farbreagenz zugegeben und inkubiert. Im letzten Schritt wird die Reaktion durch Zugabe der Stopp-Lösung beendet.

Alternativ kann auch eine gemeinsame Inkubation von Kalibratoren bzw. Proben mit dem Kombinationskonjugat erfolgen. Allerdings ist die Meerrettich-Peroxidase nicht stabil gegenüber der Cocktail Extraktionslösung in den Proben. Daher müssen die Prolamin-, LMW- und HMW-Fraktion in diesem Fall anders konjugiert werden, z. B. an Biotin. Nach einem weiteren Waschschritt erfolgt die Detektion über Meerrettich-Peroxidase gekoppeltes Streptavidin. Nach einem erneuten Waschschritt wird das Farbreagenz zugegeben und inkubiert. Im letzten Schritt wird die Reaktion durch Zugabe der Stopp-Lösung beendet.

Bei einem *direkten ELISA* wird eine unbeschichtete Mikrotiterplatte verwendet. Der Kalibrator ist identisch mit dem beim Sandwich-ELISA-Verfahren. Die Herstellung sowie die Einstellung der Einzelkonjugate und des Kombinationskonjugates erfolgen wie beim Sandwich-ELISA-Verfahren. Die Probenextraktion ist identisch mit der beim Sandwich-ELISA-Verfahren.

Im ersten Schritt der ELISA-Testdurchführung wird die unbeschichtete Mikrotiterplatte mit den Kalibratoren bzw. den Proben inkubiert. Nach einem Waschschritt wird als nächstes das Kombinationskonjugat zugegeben und inkubiert. Nach einem erneuten Waschschritt wird das Farbreagenz zugegeben und inkubiert. Im letzten Schritt wird die Reaktion durch Zugabe der Stopp-Lösung beendet.

### Herstellung des Antikörper-Kombinationskonjugats:

Für das Ausführungsbeispiel der Bestimmung des Gesamt-Glutengehaltes aus Weizenhaltigen Proben, werden als Prolamin-Detektionsantiköper ein Gliadin-Antikörper gegen das Epitop QQPFP eingesetzt und als Glutelin-Detektionsantikörper ein Antikörper gegen die LMW-Fraktion extrahiert aus Weizenmehl und ein Antikörper gegen HMW mit der Epitoperkennungssequenz GYYPTS eingesetzt.

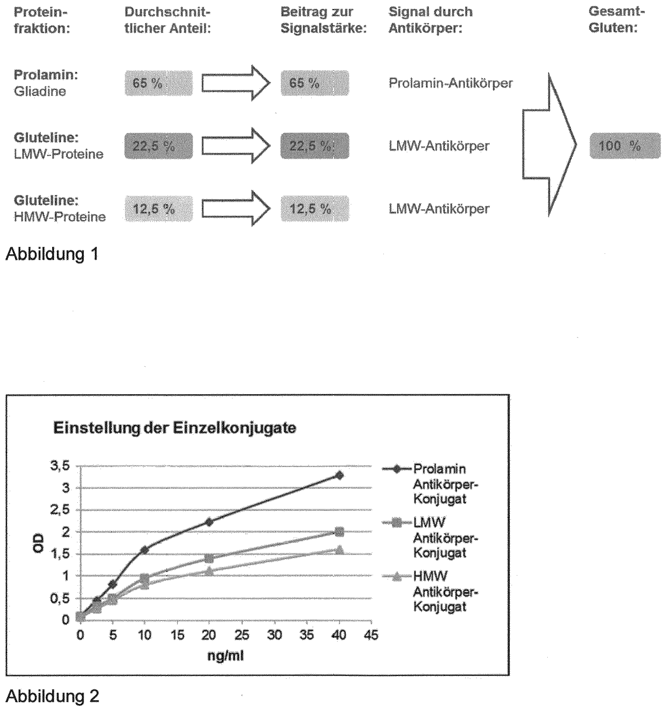

Die verschiedenen Detektionsantikörper werden unter Einsatz kommerzieller Konjugationsreagenzien (z. B. Pierce™ Conjugate Purification Kit Katalognummer 44920) separat an Meerrettich-Peroxidase gekoppelt. Anschließend werden die so erhaltenen Antikörperkonjugate einzeln in der Weise verdünnt, dass ihr Beitrag zur Gesamtsignalstärke dem aus der Literatur bekanntem Anteil der Fraktion, die sie detektieren, in Weizen entspricht (siehe Abbildung 1).

Tabelle 1 zeigt das Beispiel einer solchen Einstellung. Hierzu werden die spezifischen Antikörperkonjugate zunächst einzeln mit der oben genannten beschichteten Mikrotiterplatte getestet. Ein Weizengesamtglutenextrakt wird auf drei verschiedene Konzentrationen verdünnt, bei denen jeweils dieselbe Menge an entweder Prolamin-, LMW- oder HMW-Proteine enthalten sind. So enthält ein Gesamtglutenextrakt mit der Konzentration 15,5 ng/ml Gesamtglutenprotein umgerechnet 10 ng/ml Gliadin. Entsprechend enthält ein Gesamtglutenextrakt mit der Konzentration 44,5 ng/ml Gesamtglutenprotein 10 ng/ml LMW-Proteine und ein Gesamtglutenextrakt mit der Konzentration 80 ng/ml 10 ng/ml HMW-Proteine (siehe Tabelle 1).

**Tabelle 1: Auswahl von Gesamtglutenkonzentrationen, bei denen die Konzentration der jeweiligen Einzelfraktionen denselben Wert besitzt. Alle Angaben in ng/ml.**

| **Gesamt Weizengluten** | **Prolamine 65% des Gesamtglutens** | **LMW 22,50% des Gesamtglutens** | **HMW 12,50% des Gesamtglutens** |
|---|---|---|---|
| 0 | 0,0 | 0,0 | 0,0 |
| 1,3 | 0,8 | 0,3 | 0,2 |
| 2,5 | 1,6 | 0,6 | 0,3 |
| 5,0 | 3,3 | 1,1 | 0,6 |
| 10,0 | 6,5 | 2,3 | 1,3 |
| **15,5** | **10,1** | 3,5 | 1,9 |
| 20,0 | 13,0 | 4,5 | 2,5 |
| 40,0 | 26,0 | 9,0 | 5,0 |
| **44,5** | 28,9 | **10,0** | 5,6 |
| **80,0** | 52,0 | 18,0 | **10,0** |

Die Gliadin-, LMW- und HMW-spezifischen Antikörperkonjugate werden nun so eingestellt d. h. derart verdünnt, dass der Gliadin-Antikörper mit dem 15,5 ng/ml Gesamtglutenextrakt dieselbe Signalstärke besitzt (im Beispiel in Tabelle 2: 2,0) wie der LMW-Antikörper mit dem 44,5 ng/ml Gesamtglutenextrakt und der HMW-Antikörper mit dem 80 ng/ml Gesamtglutenextrakt (Tabelle 2).

**Tabelle 2: Beispiel der Testung von Einzelkonjugatverdünnungen.**

| | Prolamine | LMW | HMW |
|---|---|---|---|
| Konzentration Gesamtgluten (ng/ml) | 15,5 | 44,5 | 80,0 |
| Konzentration Zielproteinfraktion (ng/ml) | 10,1 | 10,0 | 10,0 |
| Zu testendes Einzelkonjugat | Prolamine | LMW | HMW |
| OD bei Verdünnung 1 des jeweiligen Einzelkonjugates | 3,0 | 2,4 | 4,0 |
| OD bei Verdünnung 2 des jeweiligen Einzelkonjugates | 2,5 | 2 ,0 2,0 | 3,33 |
| OD bei Verdünnung 3 des jeweiligen Einzelkonjugates | 2,0 | 1,6 | 2,66 |
| OD bei Verdünnung 4 des jeweiligen Einzelkonjugates | 1,5 | 1,2 | 2,0 |
| OD bei Verdünnung 5 des jeweiligen Einzelkonjugates | 1,0 | 0,8 | 1,33 |

Abbildung 2 zeigt die Überprüfung der so erhaltenen Konjugateinstellungen der einzelnen Detektionsantikörper. Hierzu wurden Kalibrationsreihen mit Weizengesamtglutenkonzentrationen von 0 bis 80 ng/ml mit den spezifischen Einzelkonjugaten getestet.

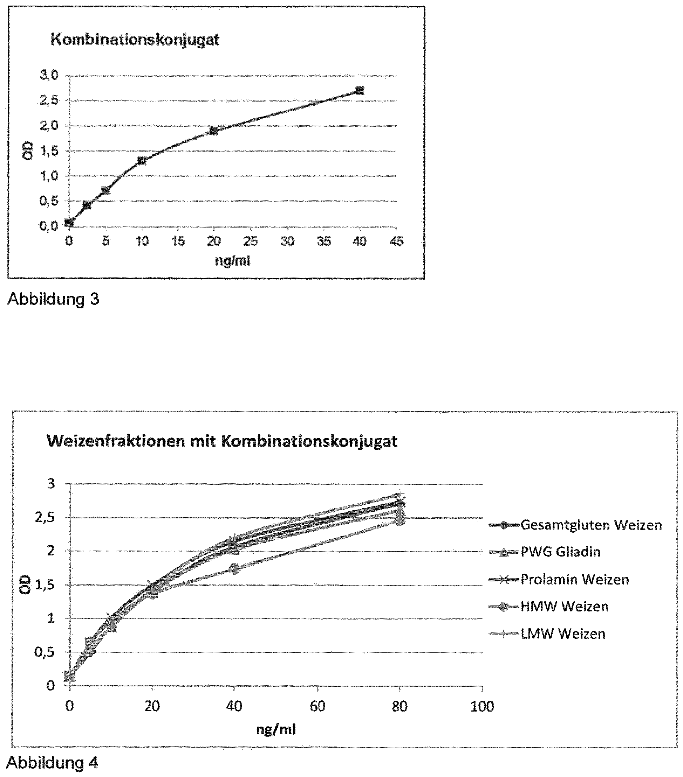

Anschließend werden die spezifischen Einzelkonjugatlösungen der Detektionsantikörper im Verhältnis 1+1+1 gemischt. Abbildung 3 zeigt die Extinktionskurve einer Weizengesamtglutenkalibrationsreihe, die mit diesem Kombinationskonjugat gemessen wurde.

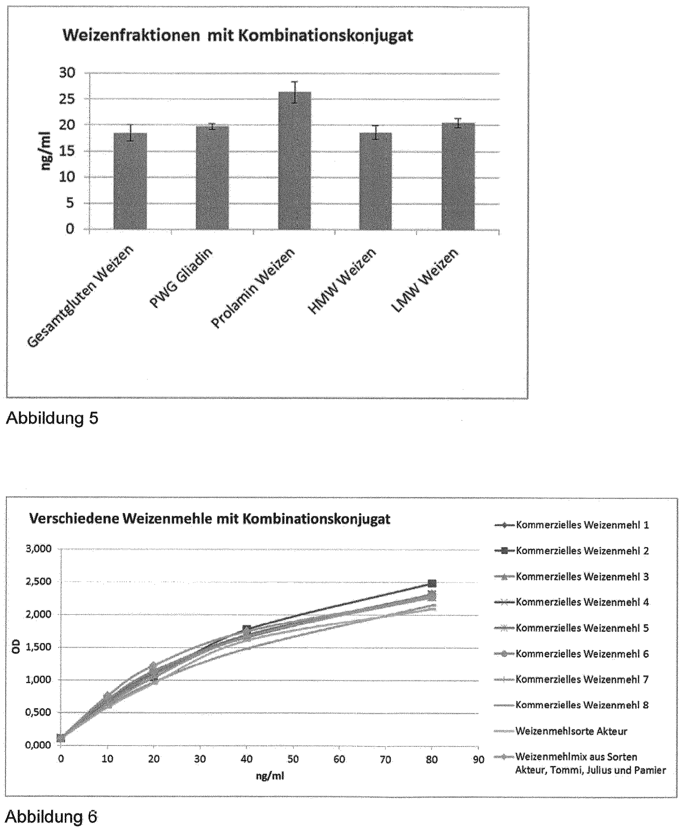

Anschließend wird das Kombinationskonjugat mit Kalibrationsreihen der Einzelfraktionen (Weizenprolamine, LMW- und HMW-Proteine) überprüft (siehe Abbildung 4 und Abbildung 5). Die Einzelfraktionen werden durch Extraktion aller Proteine aus einem Weizenmehl und anschließender Auftrennung in der HPLC erhalten (Schalk et al. 2017). Zur Verbesserung der Haltbarkeit werden die so erhaltenen Proteinisolate zur Trockne gebracht und gelagert. Zum Ansetzen der Stocklösungen werden die Fraktionen eingewogen, mittels Cocktail-Extraktionslösung (EP 1 424 345 A1) gelöst und in einem pH neutralen Phosphatpuffer auf die gewünschten Konzentrationen verdünnt.

Im Anschluss ist noch einmal ein detailliertes Ausführungsbeispiel eines Sandwich ELISAs zur quantitativen Glutenbestimmung von einer Getreide oder Lebensmittelprobe z. T. aufgeführt:
- Je 100 µl der Standardlösung bzw. der vorbereiteten Proben in Doppelbestimmung in die entsprechenden Kavitäten pipettieren und 20 min bei Raumtemperatur (20 - 25 °C) inkubieren.
- Die Kavitäten durch Ausschlagen der Flüssigkeit leeren und die Restflüssigkeit durch kräftiges Ausklopfen (dreimal hintereinander) auf saugfähigen Labortüchern entfernen. Die Kavitäten mit jeweils 250 µl Waschpuffer (PBS/Tween) waschen. Diesen Vorgang zweimal wiederholen.
- Je 100 µl des Kombinationskonjugates in die entsprechenden Kavitäten pipettieren und weitere 20 min bei Raumtemperatur (20 - 25 °C) inkubieren.
- Die Kavitäten durch Ausschlagen der Flüssigkeit leeren und die Restflüssigkeit durch kräftiges Ausklopfen (dreimal hintereinander) auf saugfähigen Labortüchern entfernen. Die Kavitäten mit jeweils 250 µl Waschpuffer waschen. Diesen Vorgang zweimal wiederholen.
- Je 100 µl einer handelsüblichen Meerrettich-Peroxidase Substrat/Chromogenlösung in die Kavitäten pipettieren. Vorsichtig manuell mischen und 10 min bei Raumtemperatur (20 - 25 °C) im Dunkeln inkubieren.
- Je 100 µl Stopp Lösung in jede Kavität pipettieren und vorsichtig manuell mischen. Die Extinktion bei 450 nm innerhalb von 30 min nach Zugabe der Stopp Lösung in einem handelsüblichen Photometer messen.

### Quantifizierung des Gesamt-Glutengehalts von verschiedenen Weizenmehlen:

Weizenmehle können sich sowohl im Gesamtproteingehalt als auch in der Zusammensetzung der Glutenproteine deutlich unterscheiden (Hajas et al. 2017). Aufgrund der Einstellung der Einzelkonjugate sollten Unterschiede in der Zusammensetzung der Glutenproteine keinen Einfluss auf die Messung haben, da dieselbe Menge an Einzelfraktionprotein zu einem vergleichbaren Signal führt, egal um welche Weizenproteinfraktion es sich handelt (siehe Abbildung 4). Dadurch, dass alle Zöliakie-relevanten Glutenproteinfraktionen bestimmt werden, wird im Gegensatz zu herkömmlichen Testmethoden (siehe Stand der Technik) der Gesamtglutengehalt bei unterschiedlicher Glutenproteinzusammensetzung nicht verfälscht.

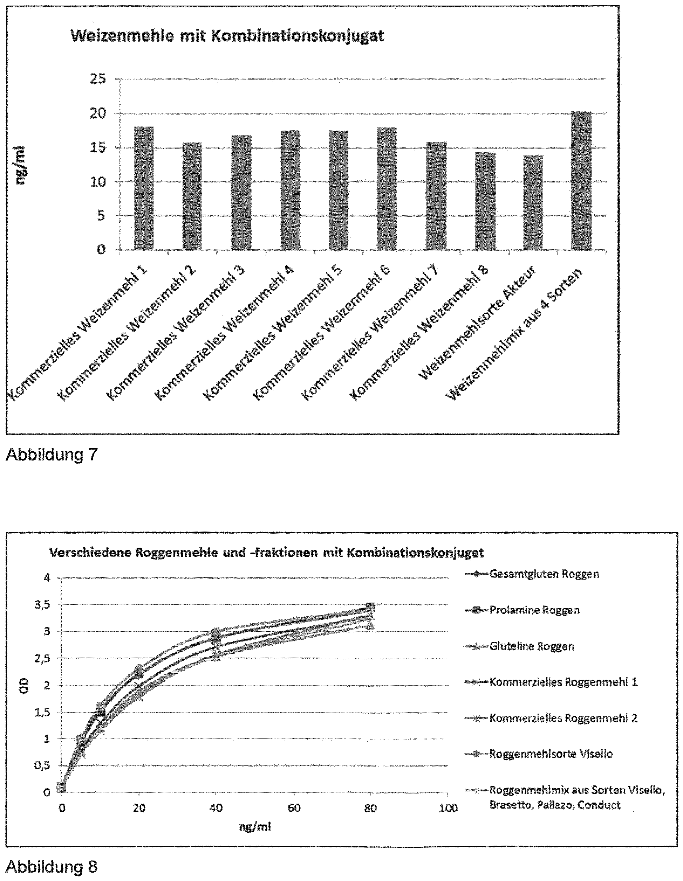

Für die Bestimmung des Gesamt-Glutengehalts von verschiedenen Weizenmehlen wurden neun Weizenmehle und ein Weizenmehlmix mit der Cocktail-Extraktionslösung aus EP 1 424 345 A1 extrahiert. Die Extrakte wurden im Extraktionsmedium und einem pH neutralen Probenverdünnungspuffer auf die Konzentrationen 0; 5; 10; 20; 40 und 80 ng/ml Gesamtglutenprotein verdünnt. Die Glutenproteingehalte der verschiedenen Mehle waren zuvor mittels HPLC ermittelt worden (Schalk et al. 2017). Abbildungen 6 und 7 zeigen, dass alle Weizenmehle innerhalb akzeptabler Schwankungsbereiten gefunden werden, insbesondere wenn der extrem hohe Verdünnungsfaktor berücksichtigt wird, der für die Messung der Extrakte erforderlich war.

### Quantitative Bestimmung des Gesamt-Glutengehalts in anderen Getreiden

Auch andere Getreidemehle können sich sowohl im Gesamtproteingehalt als auch in der Zusammensetzung der Glutenproteine deutlich unterscheiden. Die Gesamtreaktivität des Messsystems hängt dabei von der Stärke der einzelnen Kreuzreaktivitäten der eingesetzten Antikörper zu den anderen Getreiden ab. So zeigen z. B. die Prolamine aus Weizen, Roggen und Gerste sehr große Sequenzhomologien, so dass hier mit einem hohen Level an Kreuzreaktivität des Prolaminantikörpers zu rechnen ist. Weiterhin zeigen die HMW-Proteine aus Weizen und Roggen große Sequenzhomologien, so dass auch hier von einer hohen Kreuzreaktivität des Weizen-HMW-Antikörpers auszugehen ist. Je nachdem, welche Sequenzen genau die Prolamin- und HMW-Antikörper erkennen und wie häufig diese Sequenzen in den Getreiden vorkommen, ist auch mit einer Reaktivität der Antikörper zu rechnen, die die Reaktivität gegenüber Weizen übersteigt. LMW-Proteine haben keine homologen Proteine in Roggen und Gerste, so dass hier nicht mit einer Kreuzreaktivität zu rechnen ist. Die Gerstengluteline besitzen kaum Homologien zu Weizen- und Roggenproteinen, so dass eine Detektion dieser Fraktion mit Antikörpern gegen Prolamine, HMW- und LMW Proteine nicht wahrscheinlich ist.

Für die Überprüfung der Reaktivität gegenüber Roggen- und Gerstenmehlen wurden verschiedene kommerzielle Mehle und reine Sorten mit der Cocktail-Extraktionslösung aus EP 1 424 345 A1 extrahiert. Die Extrakte wurden im Extraktionsmedium und einem pH neutralen Probenverdünnungspuffer auf die Konzentrationen 0; 5; 10; 20; 40 und 80 ng/ml Gesamtglutenprotein verdünnt. Die Glutenproteingehalte der verschiedenen Mehle waren zuvor mittels HPLC ermittelt worden (Schalk et al. 2017).

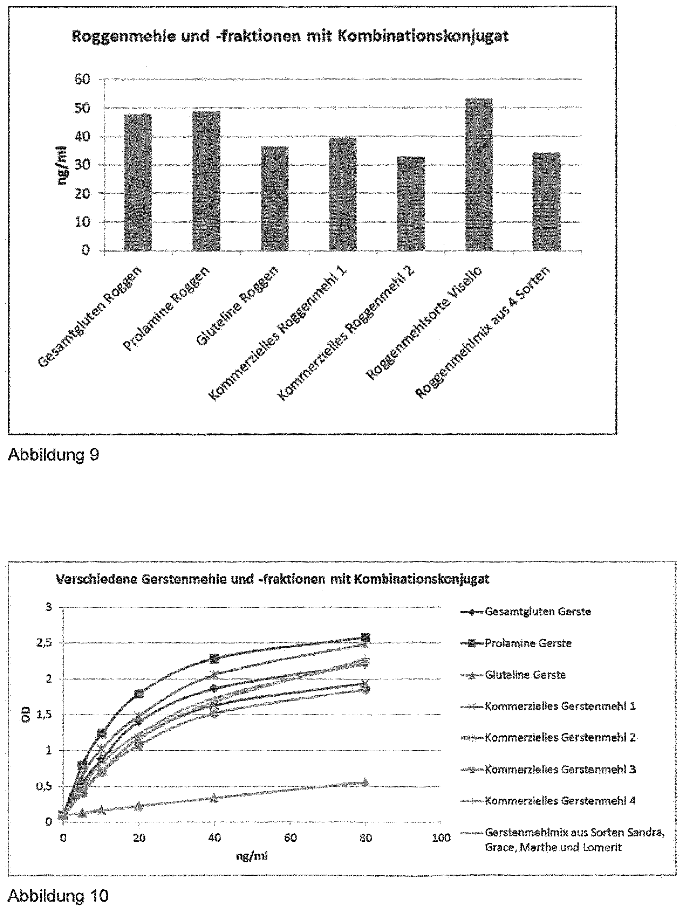

Abbildung 8 zeigt, dass alle Roggenmehle relativ ähnlich detektiert werden. Bei einer Berechnung der gemessenen Konzentrationen zeigt sich, dass alle Roggenmehle überbestimmt werden (Abbildung 9). Dies liegt in der erhöhten Reaktivität des eingesetzten Prolaminantikörpers gegenüber Roggenprolaminen begründet.

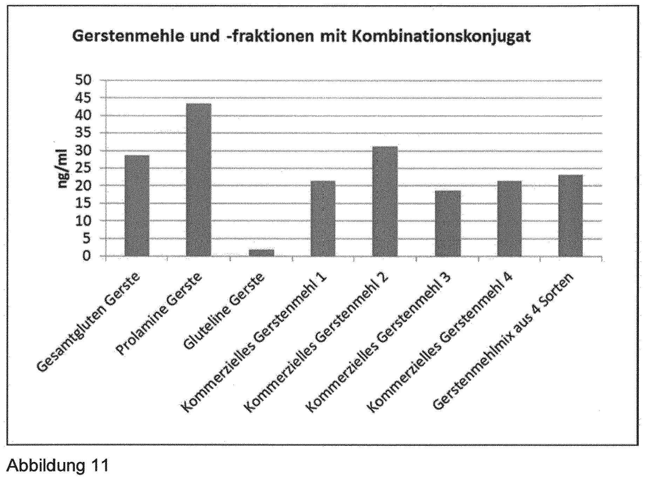

Im Gegensatz zu den Weizen- und Roggenmehlen zeigen die Gerstenmehle eine größere Schwankungsbreite (Abbildungen 10 und 11). Dies liegt vermutlich daran, dass die Gerstenfraktionen im Gegensatz zu den Weizen- und Roggenfraktionen unterschiedlich detektiert werden. Wie zu erwarten war, wird die Gerstenglutelinfraktion von keinem der eingesetzten Antikörper nennenswert detektiert, während die Prolamine sehr gut detektiert werden. Entsprechend würden geringe Verschiebungen in den Proteinfraktionen bei den verschiedenen Mehlen zu deutlichen Unterschieden führen.

Bei einer generellen Über- oder Unterbestimmung von Getreiden gegenüber einem Weizenkalibratormaterial kann zur genaueren Glutengehaltsbestimmung ein Kalibrator mit Material aus dem entsprechenden anderen Getreide eingesetzt werden.

Das beanspruchte Verfahren ermöglicht durch die Verwendung des erfindungsgemäßen Kombinationskonjugates die gleichzeitige Bestimmung von Prolamin und Glutelinproteinen und erlaubt damit erstmalig eine Quantifizierung des tatsächlichen Gesamt-Glutengehaltes. Dieses Verfahren einer gemeinsamen Bestimmung von Prolamin und Glutelinen ist sehr viel genauer als die bisher bekannten Methoden, bei denen nur der Prolamin-Gehalt bestimmt wird, aus dem anschließend wiederum der Gesamt-Glutengehalt berechnet wird. Die ebenfalls Zöliakie-relevanten Gluteline wurden bisher nicht mit bestimmt, was in der Vergangenheit zu falschen Ergebnissen führen konnte. Das beanspruchte Verfahren schließt diese Lücke.

Darüber hinaus ist das erfindungsgemäße Verfahren nicht nur zur Bestimmung des Gesamt-Glutenhaltes weizenhaltiger Lebensmittelproben geeignet, sondern auch für die Bestimmung des Gesamt-Glutengehaltes anderer Triticum-Spezies-, und/oder Roggen- und/oder Gerste-haltigen Lebensmittelproben.

### Legende zu den Abbildungen

- Abb. 1: Übersicht über die Einstellung der spezifischen Einzelkonjugate der Detektionsantikörper
- Abb. 2: Testung der Einzelkonjugate mit Weizengesamtglutenextrakt nach der Einstellung der Einzelkonjugate nach Tabelle 1
- Abb. 3: Testung des Kombinationskonjugates mit Weizengesamtglutenextrakt nach der Einstellung der Einzelkonjugate entsprechend Tabelle 1
- Abb. 4: Testung der Reaktivität des Kombinationskonjugates gegenüber den verschiedenen Weizenmehlfraktionen
- Abb. 5: Testung der Reaktivität des Kombinationskonjugates gegenüber den verschiedenen Weizenmehlfraktionen. Gemessene Konzentrationen für verschiedene Weizenmehlfraktionen bei einem Zielwert von 20 ng/ml
- Abb. 6: Testung der Reaktivität des Kombinationskonjugates gegenüber verschiedener kommerzieller Weizenmehle und einiger reiner Weizensorten, deren Glutenproteingehalt jeweils mittels HPLC bestimmt wurde
- Abb. 7: Testung der Reaktivität des Kombinationskonjugates gegenüber verschiedener kommerzieller Weizenmehle und reinen Weizensorten, deren Glutenproteingehalt jeweils mittels HPLC bestimmt wurde. Gemessene Konzentrationen bei einem Zielwert von 20 ng/ml
- Abb. 8: Testung der Reaktivität des Kombinationskonjugates gegenüber verschiedener kommerzieller Roggenmehle und reiner Roggensorten, deren Glutenproteingehalt jeweils mittels HPLC bestimmt wurde, sowie aufgereinigter Roggenfraktionen
- Abb. 9: Testung der Reaktivität des Kombinationskonjugates gegenüber verschiedenen kommerziellen Roggenmehlen und reinen Roggensorten, deren Glutenproteingehalt jeweils mittels HPLC bestimmt wurde, sowie aufgereinigter Roggenfraktionen. Gemessene Konzentrationen bei einem Zielwert von 20 ng/ml
- Abb. 10: Testung der Reaktivität des Kombinationskonjugates gegenüber verschiedenen kommerziellen Gerstenmehlen und reinen Gerstensorten, deren Glutenproteingehalt jeweils mittels HPLC bestimmt wurde, sowie aufgereinigten Gerstenfraktionen
- Abb. 11: Testung der Reaktivität des Kombinationskonjugates gegenüber verschiedenen kommerziellen Gerstenmehlen und reinen Gerstensorten, deren Glutenproteingehalt jeweils mittels HPLC bestimmt wurde, sowie aufgereinigten Gerstenfraktionen. Gemessene Konzentrationen bei einem Zielwert von 20 ng/ml

### Literatur

Hajas, L., Scherf, K., Török, K., Bugyi, Z., Schall, E., Poms, R., Koehler, P. and Tömösközi, S. (2017) Variation in protein composition among wheat (Triticum aestivum L.) cultivars to identify cultivars suitable as reference material for wheat gluten analysis. Food Chemistry.
Schalk, K., Lexhaller, B., Koehler, P. and Scherf, K. (2017) Isolation and characterization of gluten protein types from wheat, rye, barley and oats for use as reference materials. PLOS One.
Tye-Din, J., Stewart, J., Dromey, J., Beissbarth, T., van Heel, D., Tatham, A., Henderson, K., Mannering, S., Gianfrani, C., Jewell, D., Hill, A., McCluskey, J., Rossjohn, J. and Anderson, R. (2010) Comprehensive, Quantitative Mapping of T Cell Epitopes in Gluten in Celiac Disease. Science Translational Medicine.
Wieser, H. and Koehler, P. (2009) Is the calculation of the gluten content by multiplying the prolamin content by factor 2 valid? European Food Research Technology
Wieser, H., Koehler, P. and Konitzer, K. (2014) Celiac Disease and Gluten. Academic Press, ISBN 978-0-12-420220-7

## Patentansprüche

1. Verfahren zur Quantifizierung von Gesamt-Gluten aus Getreide in Lebensmittelproben, bei dem eine Extraktion der Gesamt-Glutenproteine aus Lebensmittelproben und deren anschließende quantitative Konzentrationsbestimmung mittels spezifischer Affinitätsbindung erfolgt, **dadurch gekennzeichnet, dass** zur Quantifizierung des Gesamtglutengehaltes eine Kombination spezifischer Bindungsreagenzien gegen Prolamine und Gluteline eingesetzt wird.

2. Verfahren nach Anspruch 1, **dadurch gekennzeichnet, dass** als spezifische Bindungsreagenzien Antikörper, Antikörperfragmente, Nanobodies, Aptamere, spezifische Rezeptoren, T-Zellrezeptoren und/oder Molecularly Imprinted Polymers (MIP); vorzugsweise Antikörper verwendet werden.

3. Verfahren nach Anspruch 1-2, **dadurch gekennzeichnet, dass** die Bestimmung von Gesamt-Gluten mit einem Sandwich-ELISA unter Verwendung einer Mikrotiterplatte mit einer gewünschten Anzahl von Kavitäten erfolgt, bei dem die einzelnen Kavitäten mit spezifischen Fängerantikörpern vorzugsweise gegen Prolamine und gegen Gluteline beschichtet werden, die extrahierte Lebensmittelprobe oder ein Kalibrator in die Kavitäten gegeben wird, nach einer Inkubation ungebundenes Proben-Material entfernt, anschließend ein Kombinationskonjugat aus konjugierten Detektionsantikörpern gegen Prolamine und Gluteline in die Kavitäten zugegeben und durch Messung der Signalstärke der Detektionsantikörper die Gesamt-Glutenmenge bestimmt wird.

4. Verfahren nach Anspruch 1-3, **dadurch gekennzeichnet, dass** die verwendeten Fängerantikörper gegen Prolamine und Gluteline und die verwendeten Detektionsantikörper gegen Prolamine und Gluteline jeweils an unterschiedlichen Stellen der Prolamine und Gluteline binden

5. Verfahren nach Anspruch 1-4, **dadurch gekennzeichnet, dass** die verwendeten Detektionsantikörper gegen Prolamine und Gluteline separat mit einem quantifizierbaren Marker konjugiert werden, vorzugsweise einem Reporterenzym.

6. Verfahren nach Anspruch 1-5, **dadurch gekennzeichnet, dass** die verwendeten konjugierten Detektionsantikörper gegen Prolamine und Gluteline separat derart verdünnt werden, dass ihr Beitrag zur Signalstärke in Bezug auf den Gesamt-Glutengehalt dem Anteil der Glutenprotein-Fraktion, den sie detektieren, entspricht, anschließend im Verhältnis 1:1 gemischt und als Kombinationskonjugat eingesetzt werden.

7. Verfahren nach einem der Ansprüche 1-6, **dadurch gekennzeichnet, dass** der Gesamtglutengehalt aus Weizen in Lebensmittelproben bestimmt wird, indem Fänger- und Detektionsantkörper gegen Gliadin als Vertreter der Prolamine und gegen LMW- und HMW-Glutenine als Vertreter der Gluteline verwendet werden.

8. Verfahren nach Anspruch 7, **dadurch gekennzeichnet, dass** als Prolamin-Detektionsantiköper ein Antikörper gegen das Epitop QQPFP eingesetzt wird, als LMW-Detektionsantikörper ein Antikörper gegen die LMW-Fraktion extrahiert aus Weizenmehl und als HMW-Detektionsantikörper ein Antikörper gegen das Epitop GYYPTS eingesetzt wird.

9. Verfahren nach einem der Ansprüche 1-8 **dadurch gekennzeichnet, dass** ein Kalibrator mit Glutenproteinen aus Weizen, und/oder anderen Triticum-Spezies, und/oder Roggen und/oder Gerste verwendet wird.

10. Verwendung eines Verfahrens gemäß den Ansprüchen 1-9 zur Quantifizierung von Gesamtglutenproteinen aus Weizen-, und/oder anderen Triticum-Spezies- und/oder Roggen- und/oder Gerste-haltigen Lebensmittelproben.

11. Testkit zur Quantifizierung von Gesamtgluten aus Weizen, und/oder Triticum-Spezies, und/oder Gerste und/oder Roggen in Lebensmittelproben, umfassend eine Mikrotiterplatte beschichtet mit Fängerantikörpern gegen Gliadin-, LMW- und HMW-Proteine, Weizenmehlextrakt als Kalibrator, ein Kombinationskonjugat mit Meerettichperoxidase-konjugierten Detektionsantikörpern gegen Gliadin-, LMW- und HMW-Proteine, die derart verdünnt sind, dass ihr jeweiliger Beitrag zur Signalstärke in Bezug auf den Gesamt-Glutengehalt dem Anteil der Glutenprotein-Fraktion, den sie detektieren, entspricht, Farbreagenz (Substrat), Stopplösung, Probenverdünnungspuffer und Waschpuffer.
